# EUROPEAN PATENT APPLICATION

(11) **EP 4 717 184 A1**
(43) Date of publication of application: **01.04.2026**
(21) Application number: 25197540.5
(22) Date of filing: 22.08.2025
(51) Int. Cl.: A61B 8/08, A61B 8/00, G06T 7/12

(54) **ULTRASOUND DIAGNOSTIC APPARATUS AND METHOD OF CONTROLLING ULTRASOUND DIAGNOSTIC APPARATUS**

(30) Priority: 26.09.2024 JP 2024167427
(71) Applicant: FUJIFILM Corporation, Tokyo 106-8620 (JP)
(72) Inventor: TAKASHIMA, Kaito, Kaisei-machi (JP)
(74) Representative: Meissner Bolte Partnerschaft mbB

(57) **Abstract**

Provided are an ultrasound diagnostic apparatus and a method of controlling an ultrasound diagnostic apparatus that can provide support for accurate measurement of cardiac function with a simple apparatus configuration.

An ultrasound diagnostic apparatus includes: an image acquisition unit that acquires ultrasound images of a plurality of frames in which a heart of a subject is imaged using an ultrasound probe; a contour extraction unit that extracts a contour of a cardiac chamber from each of the ultrasound images of the plurality of frames; a contour correction unit that performs correction processing on a plurality of contours of the cardiac chamber extracted from the ultrasound images of the plurality of frames such that the plurality of contours of the cardiac chamber smoothly change in time series; and a display controller that displays the ultrasound image on which the contour of the cardiac chamber extracted for each frame is superimposed, on a monitor in real time, and that displays the ultrasound image on which the contour of the cardiac chamber on which the correction processing is performed is superimposed, on the monitor.

## Description

### BACKGROUND OF THE INVENTION

### 1. Field of the Invention

The present invention relates to an ultrasound diagnostic apparatus that captures an ultrasound image of a heart of a subject and a method of controlling the ultrasound diagnostic apparatus.

### 2. Description of the Related Art

In the related art, an ultrasound image representing a tomographic plane of a heart of a subject is captured using a so-called ultrasound diagnostic apparatus, and the ultrasound image is used to measure cardiac functions such as a left ventricular ejection fraction (LVEF), a left ventricular end-diastolic volume, a left ventricular end-systolic volume, E/e' which is an indicator representing a left ventricular diastolic function, a mitral annular plane systolic excursion (MAPSE), and a tricuspid annular plane systolic excursion (TAPSE). The measurement of the cardiac function may be performed, for example, in medical settings, and particularly in this case, accurate measurement is required.

Therefore, for example, a technology as disclosed in JP2017-159037A has been developed so that the measurement of the cardiac function can be accurately performed. JP2017-159037A discloses an apparatus that selects an ultrasound image corresponding to an end-diastolic phase and an end-systolic phase of the heart with reference to data in which an ultrasound image, a time of ultrasound scanning, and an electrocardiographic waveform of the subject are associated with each other, extracts a contour of a cardiac chamber in the selected ultrasound image, and calculates at least one of a volume of the cardiac chamber or an ejection fraction using information on the extracted contour.

### SUMMARY OF THE INVENTION

However, in JP2017-159037A, it is necessary to acquire the electrocardiographic waveform of the subject by an electrocardiograph in order to associate the ultrasound image and the time of the ultrasound scanning with the electrocardiographic waveform of the subject. As described above, in order to accurately measure the cardiac function in the technology of JP2017-159037A, a complicated apparatus configuration is required.

The present invention has been made in order to solve such a problem in the related art, and an object of the present invention is to provide an ultrasound diagnostic apparatus and a method of controlling an ultrasound diagnostic apparatus, which can provide support for accurate measurement of a cardiac function with a simple apparatus configuration.

The object described above can be achieved with the following configurations.
[1] An ultrasound diagnostic apparatus comprising: an ultrasound probe; a monitor; an image acquisition unit that acquires ultrasound images of a plurality of frames as a moving image in which a heart of a subject is imaged, by transmitting and receiving ultrasound beams using the ultrasound probe; a contour extraction unit that extracts a contour of a cardiac chamber from each of the ultrasound images of the plurality of frames acquired by the image acquisition unit; a contour correction unit that performs correction processing on a plurality of contours of the cardiac chamber extracted by the contour extraction unit from the ultrasound images of the plurality of frames such that the plurality of contours of the cardiac chamber smoothly change in time series; and a display controller that displays the ultrasound image on which the contour of the cardiac chamber extracted by the contour extraction unit for each frame is superimposed, on the monitor in real time, and that displays the ultrasound image on which the contour of the cardiac chamber on which the correction processing is performed by the contour correction unit is superimposed, on the monitor.
[2] The ultrasound diagnostic apparatus according to [1], further comprising: a measurement target extraction unit that extracts the ultrasound image, which is a target for measurement, from among the ultrasound images of the plurality of frames based on the contour of the cardiac chamber extracted by the contour extraction unit.
[3] The ultrasound diagnostic apparatus according to [1] or [2], in which the contour correction unit corrects the contour of the cardiac chamber extracted by the contour extraction unit such that consistency with the contour of the cardiac chamber in preceding and succeeding frames is ensured.
[4] The ultrasound diagnostic apparatus according to [1] or [2], in which the contour correction unit corrects the contour of the cardiac chamber based on positional information of a characteristic structure of the cardiac chamber.
[5] The ultrasound diagnostic apparatus according to [1] or [2], in which the contour correction unit acquires cardiac phase information based on the contour of the cardiac chamber extracted by the contour extraction unit, and corrects the contour of the cardiac chamber by referring to the cardiac phase information such that positions of the contour of the cardiac chamber corresponding to a plurality of ultrasound images having the same phase in different cardiac cycles are aligned with each other.
[6] The ultrasound diagnostic apparatus according to [1] or [2], in which the contour correction unit calculates an edge line at which a brightness difference is maximized in a vicinity of the contour of the cardiac chamber extracted by the contour extraction unit, and corrects the contour of the cardiac chamber such that the contour of the cardiac chamber is aligned with the edge line.
[7] The ultrasound diagnostic apparatus according to any one of [1] to [6], in which the display controller displays a portion of the ultrasound image related to correction performed by the contour correction unit in an enhanced manner.
[8] The ultrasound diagnostic apparatus according to any one of [1] to [6], further comprising: an image selection unit that selects the ultrasound image, which is a target for correction of the contour of the cardiac chamber via the contour correction unit, from among the ultrasound images of the plurality of frames.
[9] The ultrasound diagnostic apparatus according to [8], further comprising: an input device for a user to perform an input operation, in which the image selection unit selects the ultrasound image selected by the user via the input device as the target for correction.
[10] The ultrasound diagnostic apparatus according to [9], in which in a case in which the user selects a new ultrasound image as the target for correction via the input device after the display controller displays the ultrasound image on which the corrected contour of the cardiac chamber is superimposed, on the monitor, the contour correction unit corrects the contour of the cardiac chamber again.
[11] The ultrasound diagnostic apparatus according to [8], in which the image selection unit acquires cardiac phase information based on the contour of the cardiac chamber extracted by the contour extraction unit, and selects the ultrasound image, which is the target for correction, based on the acquired cardiac phase information.
[12] The ultrasound diagnostic apparatus according to [2], further comprising: a measurement unit that measures a cardiac function using the ultrasound image which is the target for measurement extracted by the measurement target extraction unit and on which the contour of the cardiac chamber corrected by the contour correction unit is superimposed.
[13] The ultrasound diagnostic apparatus according to [12], in which the display controller displays a measurement result of the measurement unit on the monitor together with the ultrasound image on which the contour of the cardiac chamber extracted by the contour extraction unit is superimposed.
[14] The ultrasound diagnostic apparatus according to any one of [1] to [13], in which the contour correction unit corrects the extracted contour of the cardiac chamber each time the contour of the cardiac chamber is extracted by the contour extraction unit.
[15] A method of controlling an ultrasound diagnostic apparatus, the method comprising: acquiring ultrasound images of a plurality of frames as a moving image in which a heart of a subject is imaged, by transmitting and receiving ultrasound beams using an ultrasound probe; extracting a contour of a cardiac chamber from each of the acquired ultrasound images of the plurality of frames; performing correction processing on a plurality of contours of the cardiac chamber extracted from the ultrasound images of the plurality of frames such that the plurality of contours of the cardiac chamber smoothly change in time series; and displaying the ultrasound image on which the contour of the cardiac chamber extracted for each frame is superimposed, on a monitor in real time, and displaying the ultrasound image on which the contour of the cardiac chamber on which the correction processing is performed is superimposed, on the monitor.

The aspect of the present invention provides the ultrasound diagnostic apparatus comprising: the ultrasound probe; the monitor; the image acquisition unit that acquires the ultrasound images of the plurality of frames as the moving image in which the heart of the subject is imaged by transmitting and receiving the ultrasound beams using the ultrasound probe; the contour extraction unit that extracts the contour of the cardiac chamber from each of the ultrasound images of the plurality of frames acquired by the image acquisition unit; the contour correction unit that performs the correction processing on the plurality of contours of the cardiac chamber extracted by the contour extraction unit from the ultrasound images of the plurality of frames such that the plurality of contours of the cardiac chamber smoothly change in time series; and the display controller that displays the ultrasound image on which the contour of the cardiac chamber extracted by the contour extraction unit for each frame is superimposed, on the monitor in real time, and that displays the ultrasound image on which the contour of the cardiac chamber on which the correction processing is performed by the contour correction unit is superimposed, on the monitor, so that it is possible to provide support for accurate measurement of the cardiac function with a simple apparatus configuration.

### BRIEF DESCRIPTION OF THE DRAWINGS

FIG. 1 is a block diagram showing a configuration of an ultrasound diagnostic apparatus according to Embodiment 1 of the present invention.
FIG. 2 is a block diagram showing an internal configuration of a transmission/reception circuit in Embodiment 1 of the present invention.
FIG. 3 is a block diagram showing an internal configuration of an image generation unit in Embodiment 1 of the present invention.
FIG. 4 is a schematic diagram showing an example of an ultrasound image representing an apical four-chamber cross section.
FIG. 5 is a diagram showing an example of a contour of a cardiac chamber extracted from each of ultrasound images of three consecutive frames.
FIG. 6 is a diagram showing contours of the cardiac chamber extracted from the ultrasound images of the three consecutive frames in a superimposed manner.
FIG. 7 is a diagram showing an example of contours of the cardiac chamber extracted from the ultrasound images of the three frames having the same phase in different cardiac cycles.
FIG. 8 is a diagram showing the contours of the cardiac chamber extracted from the ultrasound images of the three frames having the same phase in different cardiac cycles in a superimposed manner.
FIG. 9 is a diagram showing an example of a position of a characteristic structure in the cardiac chamber.
FIG. 10 is a diagram showing examples of the extracted contour of the cardiac chamber and an edge line at which a brightness difference of the ultrasound image is maximized in the vicinity of the contour.
FIG. 11 is a diagram showing an example in which the ultrasound image including the contour of the cardiac chamber before correction and the ultrasound image including the contour of the cardiac chamber after correction are displayed side by side.
FIG. 12 is a diagram showing an example in which a correction portion is displayed in an enhanced manner in the ultrasound image including the contour of the cardiac chamber after correction.
FIG. 13 is a diagram showing an example in which the ultrasound image in which the contour of the cardiac chamber is corrected and a correction reference image used for correcting the contour of the cardiac chamber are displayed.
FIG. 14 is a flowchart showing an operation of the ultrasound diagnostic apparatus according to Embodiment 1 of the present invention.
FIG. 15 is a flowchart showing an operation of an ultrasound diagnostic apparatus according to Embodiment 2 of the present invention.
FIG. 16 is a block diagram showing a configuration of the ultrasound diagnostic apparatus according to Embodiment 2 of the present invention.

### DESCRIPTION OF THE PREFERRED EMBODIMENTS

Hereinafter, embodiments of the present invention will be described with reference to the accompanying drawings.

The following configuration requirements are described based on a representative embodiment of the present invention, but the present invention is not limited to the embodiment.

In the present specification, a numerical range represented by "to" means a range including numerical values described before and after "to", both ends inclusive, as a lower limit value and an upper limit value.

In the present specification, "the same" includes an error range generally allowed in the technical field.

### Embodiment 1

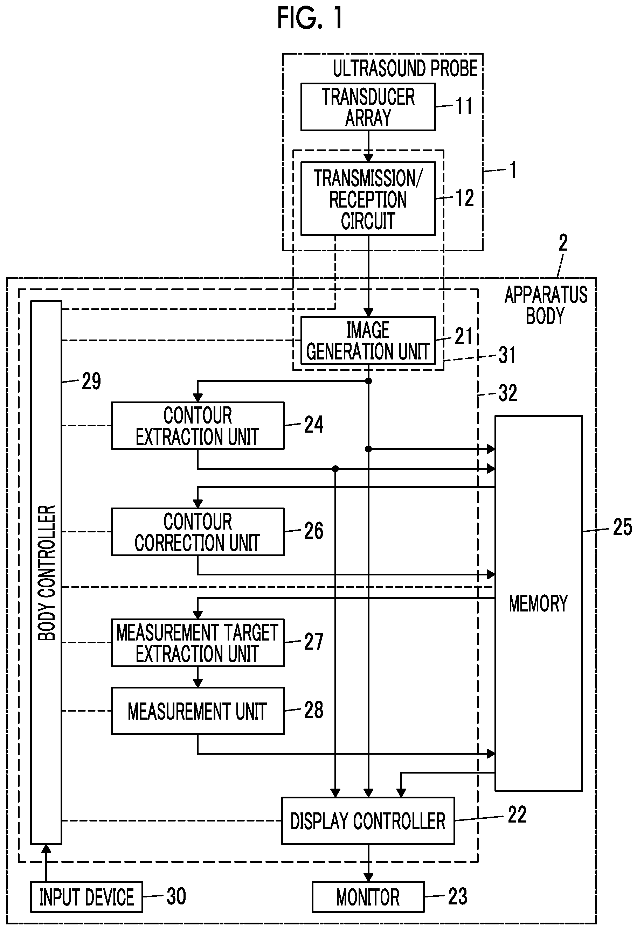

FIG. 1 shows a configuration of an ultrasound diagnostic apparatus according to Embodiment 1 of the present invention. The ultrasound diagnostic apparatus comprises an ultrasound probe 1 and an apparatus body 2 that are connected to each other by so-called wired communication or so-called wireless communication.

The ultrasound probe 1 comprises a transducer array 11 and a transmission/reception circuit 12 connected to the transducer array 11.

The apparatus body 2 comprises an image generation unit 21 connected to the transmission/reception circuit 12. In the apparatus body 2, a display controller 22 and a monitor 23 are sequentially connected to the image generation unit 21. A contour extraction unit 24 and a memory 25 are connected to the image generation unit 21. The display controller 22 and the memory 25 are connected to the contour extraction unit 24. A contour correction unit 26 is connected to the memory 25. Further, a measurement target extraction unit 27 is connected to the memory 25. A measurement unit 28 is connected to the measurement target extraction unit 27. The measurement unit 28 is connected to the memory 25. The memory 25 is connected to the display controller 22. In addition, a body controller 29 is connected to the transmission/reception circuit 12, the image generation unit 21, the display controller 22, the contour extraction unit 24, the memory 25, the contour correction unit 26, the measurement target extraction unit 27, and the measurement unit 28. An input device 30 is connected to the body controller 29.

The transmission/reception circuit 12 and the image generation unit 21 constitute an image acquisition unit 31. In addition, the image generation unit 21, the display controller 22, the contour extraction unit 24, the contour correction unit 26, the measurement target extraction unit 27, the measurement unit 28, and the body controller 29 constitute a processor 32 for the apparatus body 2.

The transducer array 11 of the ultrasound probe 1 includes a plurality of ultrasound transducers that are one-dimensionally or two-dimensionally arranged. In accordance with a drive signal supplied from the transmission/reception circuit 12, each of the ultrasound transducers transmits ultrasound and receives an ultrasound echo from a subject to output a signal based on the ultrasound echo. Each ultrasound transducer is configured by, for example, forming electrodes at both ends of a piezoelectric material consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), and the like.

The image acquisition unit 31, which is composed of the transmission/reception circuit 12 and the image generation unit 21, acquires ultrasound images of a plurality of frames as a moving image in which a heart of the subject is imaged, by transmitting and receiving ultrasound beams using the ultrasound probe 1.

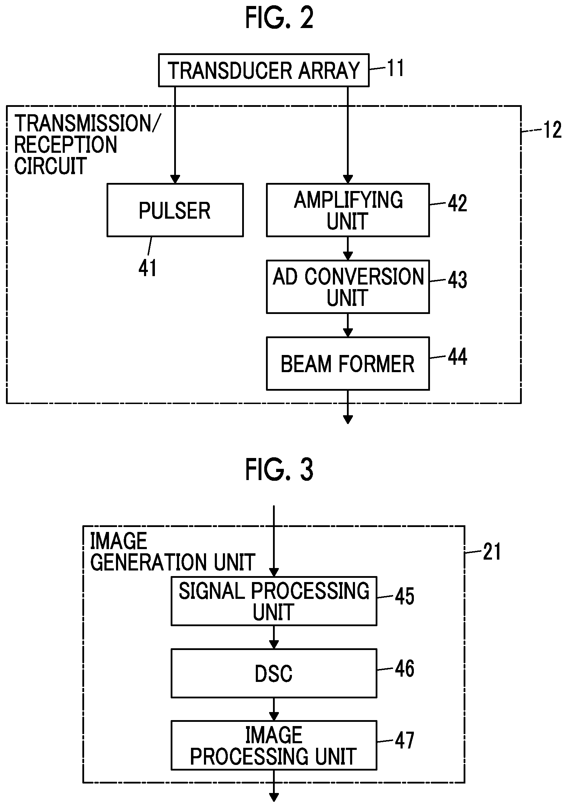

Under the control of the body controller 29, the transmission/reception circuit 12 causes the transducer array 11 to transmit the ultrasound and generates a sound ray signal based on a reception signal acquired by the transducer array 11. As shown in FIG. 2, the transmission/reception circuit 12 includes a pulser 41 connected to the transducer array 11, and an amplifying unit 42, an analog-to-digital (AD) conversion unit 43, and a beam former 44 that are sequentially connected in series to the transducer array 11.

The pulser 41 includes, for example, a plurality of pulse generators, and the pulser 41 adjusts an amount of delay of each drive signal such that the ultrasound transmitted from the plurality of ultrasound transducers of the transducer array 11 form the ultrasound beam, based on a transmission delay pattern selected in accordance with a control signal from the body controller 29, and supplies the adjusted signal to the plurality of ultrasound transducers. As described above, in a case in which a pulsed or continuous wave-like voltage is applied to the electrodes of the ultrasound transducer of the transducer array 11, the piezoelectric material expands and contracts to generate pulsed or continuous wave-like ultrasound from each of the ultrasound transducers, whereby the ultrasound beam is formed from the combined wave of the ultrasound.

The transmitted ultrasound beam is, for example, reflected by a target such as a part of the subject and propagates toward the transducer array 11 of the ultrasound probe 1. The ultrasound echo propagating toward the transducer array 11 as described above is received by each of the ultrasound transducers constituting the transducer array 11. In such a case, each of the ultrasound transducers constituting the transducer array 11 receives the propagating ultrasound echo to expand and contract, generates the reception signal, which is an electrical signal, and outputs these reception signals to the amplifying unit 42.

The amplifying unit 42 amplifies the signal input from each of the ultrasound transducers constituting the transducer array 11, and transmits the amplified signal to the AD conversion unit 43. The AD conversion unit 43 converts the signal, which is transmitted from the amplifying unit 42, into digital reception data. The beam former 44 performs so-called reception focus processing by applying respective delays to the reception data received from the AD conversion unit 43 and then adding the delayed data together. By the reception focus processing, each reception data, which is converted by the AD conversion unit 43, is phase-added, and the sound ray signal in which the focus of the ultrasound echo is narrowed down is acquired.

As shown in FIG. 3, the image generation unit 21 has a configuration in which a signal processing unit 45, a digital scan converter (DSC) 46, and an image processing unit 47 are sequentially connected in series.

The signal processing unit 45 generates a B-mode image signal, which is tomographic image information related to tissues inside the subject, by performing, on the sound ray signal received from the transmission/reception circuit 12, correction of the attenuation due to a distance in accordance with a depth of a reflection position of the ultrasound by using a sound velocity value set by the body controller 29 and then performing envelope detection processing.

The DSC 46 converts (raster-converts) the B-mode image signal, which is generated by the signal processing unit 45, into the image signal in accordance with a normal television signal scanning method.

The image processing unit 47 performs various types of necessary image processing such as gradation processing on the B-mode image signal input from the DSC 46, and then transmits the B-mode image signal to the display controller 22, the contour extraction unit 24, and the memory 25. Hereinafter, the B-mode image signal, which is image-processed by the image processing unit 47, will be referred to as an ultrasound image.

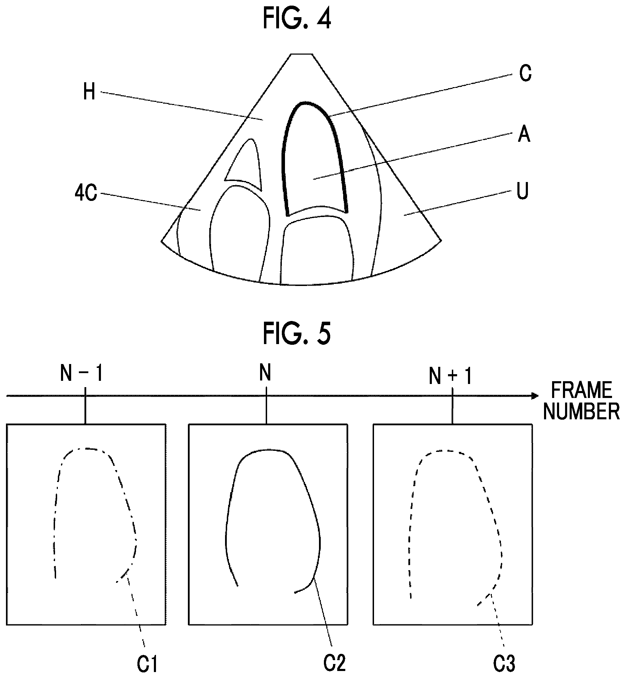

The ultrasound diagnostic apparatus according to Embodiment 1 of the present invention is used to measure cardiac functions such as a so-called left ventricular ejection fraction (LVEF), a left ventricular end-diastolic volume, a left ventricular end-systolic volume, E/e' which is an indicator indicating a left ventricular diastolic function, a mitral annular plane systolic excursion (MAPSE), and a tricuspid annular plane systolic excursion (TAPSE). In order to measure the cardiac function, for example, as shown in FIG. 4, the image acquisition unit 31 acquires the ultrasound images U of the plurality of frames representing the cross section of a cardiac chamber A, such as a so-called apical four-chamber cross section 4C of a heart H. Here, the cardiac chamber A refers to any one of a left ventricle, a left atrium, a right ventricle, or a right atrium. As the cross section of the cardiac chamber A, in addition to the apical four-chamber cross section 4C, for example, a so-called apical two-chamber cross section, apical three-chamber cross section, apical five-chamber cross section, long-axis cross section, parasternal left-ventricular long-axis cross section, parasternal left-ventricular short-axis cross section, parasternal long-axis cross section, parasternal short-axis cross section, parasternal four-chamber cross section, subcostal four-chamber cross section, subcostal short-axis cross section, and the like can be imaged.

The display controller 22 performs predetermined processing on the ultrasound image U or the like acquired by the image acquisition unit 31, and displays the processed ultrasound image U or the like on the monitor 23, under the control of the body controller 29.

The monitor 23 displays the ultrasound image U or the like under the control of the display controller 22 and includes, for example, a display device such as a liquid crystal display (LCD) or an organic electroluminescence display (organic EL display).

The memory 25 stores the ultrasound image U or the like acquired by the image acquisition unit 31 under the control of the body controller 29.

In addition, as the memory 25, for example, a recording medium such as a flash memory, a hard disk drive (HDD), a solid-state drive (SSD), a flexible disk (FD), a magneto-optical disk (MO disk), a magnetic tape (MT), a random-access memory (RAM), a compact disc (CD), a digital versatile disc (DVD), a secure digital card (SD card), or a universal serial bus memory (USB memory) can be used.

The body controller 29 controls each unit of the apparatus body 2 and the transmission/reception circuit 12 of the ultrasound probe 1 based on a control program and the like stored in advance.

The input device 30 is an input device for the user to perform an input operation, and is configured by, for example, a device such as a keyboard, a mouse, a trackball, a touchpad, and a touch sensor disposed in a state of being superimposed on the monitor 23.

The contour extraction unit 24 extracts a contour C of the cardiac chamber A for each frame, for example, as shown in FIG. 4, by performing image analysis of each of the ultrasound images U of the plurality of frames acquired by the image acquisition unit 31. In FIG. 4, the left ventricle is shown as an example of the cardiac chamber A.

The contour extraction unit 24 can extract the contour C of the cardiac chamber A from the ultrasound image U by segmentation using a learning model in so-called machine learning, which has learned a relationship between a large number of ultrasound images including the cardiac chamber A and the contour C of the cardiac chamber A. In addition, the contour extraction unit 24 can also extract the contour C of the cardiac chamber A from the ultrasound image U using a so-called template matching method of, for example, storing a plurality of template image data representing the cardiac chamber A in advance and searching for the ultrasound image U using the plurality of template image data.

The processing of extracting the contour C of the cardiac chamber A via the contour extraction unit 24 is performed each time the ultrasound image U is acquired by the image acquisition unit 31. The contour extraction unit 24 transmits information on the extracted contour C of the cardiac chamber A to the display controller 22 and the memory 25.

The memory 25 stores the ultrasound image U acquired by the image acquisition unit 31 and the information on the contour C of the cardiac chamber A extracted by the contour extraction unit 24 from the ultrasound image U in association with each other.

The display controller 22 displays the ultrasound image U on which the contour C of the cardiac chamber A is superimposed, on the monitor 23 in real time, for example, as shown in FIG. 4, based on the ultrasound image U acquired by the image acquisition unit 31 and the information on the contour C of the cardiac chamber A extracted by the contour extraction unit 24 from the ultrasound image U. Here, the ultrasound image U on which the contour C of the cardiac chamber A is superimposed refers to the ultrasound image U on which the contour C extracted by the contour extraction unit 24 is superimposed in an enhanced manner. In addition, displaying the ultrasound image U on which the contour C of the cardiac chamber A is superimposed, on the monitor 23 in real time means that the ultrasound image U on which the contour C of the cardiac chamber A is superimposed is sequentially displayed on the monitor 23 each time the ultrasound image U is acquired by the image acquisition unit 31.

The contour correction unit 26 performs correction processing on a plurality of contours C of the cardiac chamber A extracted by the contour extraction unit 24 from the ultrasound images U of the plurality of frames acquired by the image acquisition unit 31 such that the plurality of contours C of the cardiac chamber A smoothly change in time series.

In general, it is known that the contour C of the cardiac chamber A increases with the passage of time in a diastolic phase of the heart H, and the contour C of the cardiac chamber A decreases with the passage of time in a systolic phase of the heart H. Therefore, the contour correction unit 26 can perform the correction processing on the plurality of contours C of the cardiac chamber A extracted by the contour extraction unit 24 such that the consistency with the contour C of the cardiac chamber A in the preceding and succeeding frames in time series is ensured, for example, by focusing on a magnitude relationship of the contour C of the cardiac chamber A with respect to the preceding and succeeding frames in time series in the diastolic phase or the systolic phase of the heart H.

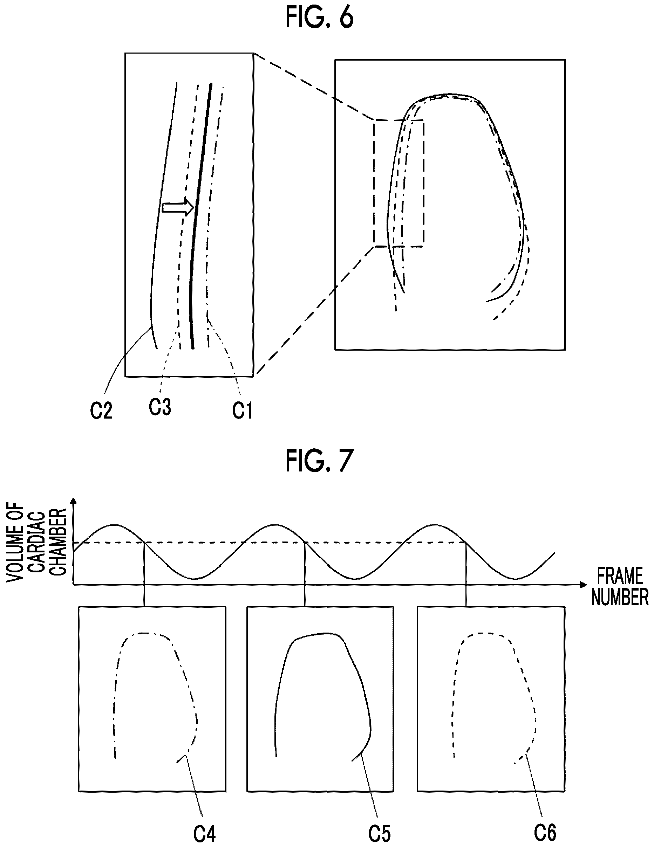

FIG. 5 schematically shows contours C1, C2, and C3 of the cardiac chamber A in the ultrasound images U of the three frames, that is, (N-1)th, Nth, and (N+1)th frames belonging to the diastolic phase of the heart H. In a case of correcting the contour C2 in the ultrasound image U of the Nth frame, the contour correction unit 26 compares the positions of the contours C1, C2, and C3 with each other, for example, as schematically shown in FIG. 6. Since the ultrasound images U of the three frames, that is, the (N-1)th, Nth, and (N+1)th frames belong to the diastolic phase, the contour C2 should be located outside the contour C1 and located inside the contour C3. Therefore, for example, as shown in FIG. 6, in a case in which the contour C2 is located outside the contour C3, the contour correction unit 26 can deform the contour C2 to be located between the contour C1 and the contour C3. In addition, although not shown, the contour correction unit 26 can deform the contour C2 to be located between the contour C1 and the contour C3 even in a case in which the contour C2 is located inside the contour C1, for example.

In addition, although not shown, in a case in which the ultrasound images U of the three frames, that is, the (N-1)th, Nth, and (N+1)th frames belong to the systolic phase of the heart H, the contour C2 should be located inside the contour C1 and located outside the contour C3. In this case, the contour correction unit 26 can deform the contour C2 to be located between the contour C1 and the contour C3 in a case in which the contour C2 is located outside the contour C1 and in a case in which the contour C2 is located inside the contour C3.

In addition, a size and a shape of the contour C of the cardiac chamber A typically change periodically in accordance with the cardiac beat. Therefore, even in a case of the different cardiac cycles, the ultrasound images U of the frames having the same phase should ideally show the contour C of the cardiac chamber A having the same size and shape. Therefore, the contour correction unit 26 can also correct the contour C of the cardiac chamber A, for example, to align the positions of the plurality of contours C of the cardiac chamber A corresponding to the plurality of ultrasound images U having the same phase in different cardiac cycles.

FIG. 7 schematically shows contours C4, C5, and C6 of the cardiac chamber A in the ultrasound images U of the three frames having the same phase in different cardiac cycles in association with the volume of the cardiac chamber A that changes in accordance with the cardiac beat. The contour correction unit 26 first acquires cardiac phase information representing a phase of each cardiac cycle based on the contour C of the cardiac chamber A extracted by the contour extraction unit 24. In this case, the contour correction unit 26 can calculate, for example, the volume of the cardiac chamber A from the area of the cardiac chamber A surrounded by the contour C shown in the ultrasound image U of each frame, to acquire a cardiac cycle number assigned to each cardiac beat for distinguishing the cardiac cycle and a frame number of the ultrasound image U having, as a starting point, the frame corresponding to the end-systolic phase or the end-diastolic phase of the heart H in each cardiac cycle, that is, the minimum value or the maximum value of the volume of the cardiac chamber A, based on a time-series change in calculated volume of the cardiac chamber A, as the cardiac phase information. Here, the contour correction unit 26 can calculate the volume of the cardiac chamber A by a known method, for example, calculating a total number of pixels surrounded by the extracted contour C.

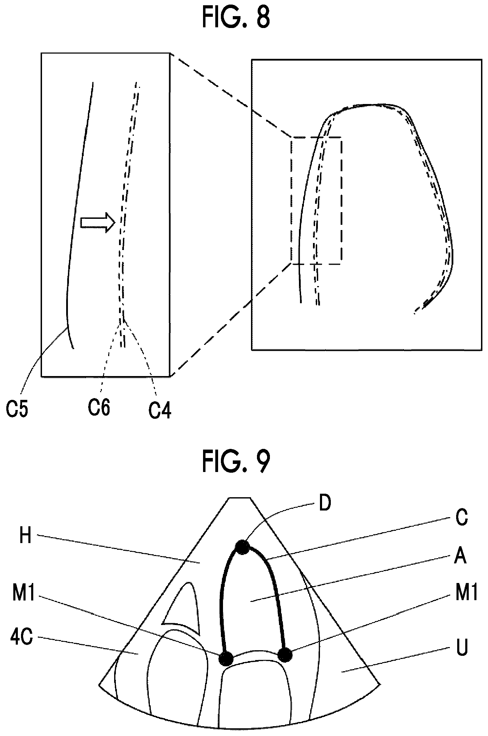

Further, the contour correction unit 26 specifies, for example, the contours C4, C5, and C6, which are shown in the plurality of ultrasound images U having the same phase in different cardiac cycles as shown in FIG. 7, by referring to the acquired cardiac phase information. In a case of correcting the contour C2, the contour correction unit 26 compares the positions of the contours C4, C5, and C6 with each other, for example, as schematically shown in FIG. 8. Since the contours C4, C5, and C6 belong to different cardiac cycles but are in the same phase, and the positions of the contours C4, C5, and C6 should ideally overlap each other. Therefore, for example, as shown in FIG. 8, in a case in which the contour C5 is separated from the contours C4 and C6, the contour correction unit 26 can deform the contour C5 to overlap the contours C4 and C6.

In addition, after acquiring the cardiac phase information, the contour correction unit 26 can also specify a characteristic structure of the cardiac chamber A, such as a so-called cardiac apex, a mitral valve, or an interventricular septum, from the ultrasound image U of each frame, and correct the contour C of the cardiac chamber A based on positional information of the specified structure. Even in a case in which the cardiac cycles are different from each other, in the same phase, the positions of the interventricular septum or an anterior wall of the left ventricle in the ultrasound image U should ideally be the same as each other. Since the interventricular septum or the anterior wall of the left ventricle is usually drawn with high brightness in the ultrasound image U, the contour correction unit 26 specifies the interventricular septum or the anterior wall of the left ventricle shown in the ultrasound image U of each frame based on the brightness information of the image. Specifically, the contour correction unit 26 can specify the interventricular septum or the anterior wall of the left ventricle by, for example, a method of using a trained model in machine learning, a method of template matching, or the like.

The contour correction unit 26 moves the entire contour C of the cardiac chamber A in the ultrasound image U of each frame such that the specified position of the interventricular septum or the anterior wall of the left ventricle matches at each phase in a plurality of cardiac cycles. Further, the contour correction unit 26 specifies a structure of interest, such as a cardiac apex D, mitral valve bases M1 and M2, or the like, of which the position is ideally not changed in the ultrasound image U due to the cardiac beat, as shown in FIG. 9, by a method of using a trained model in machine learning, a method of template matching, or the like for the ultrasound image U of the frame that is a target for correction. The contour correction unit 26 can compare the position of the structure of interest with the plurality of contours C having the same phase in the plurality of cardiac cycles, and can deform the contour C such that the position of the structure of interest is aligned with the plurality of contours C having the same phase.

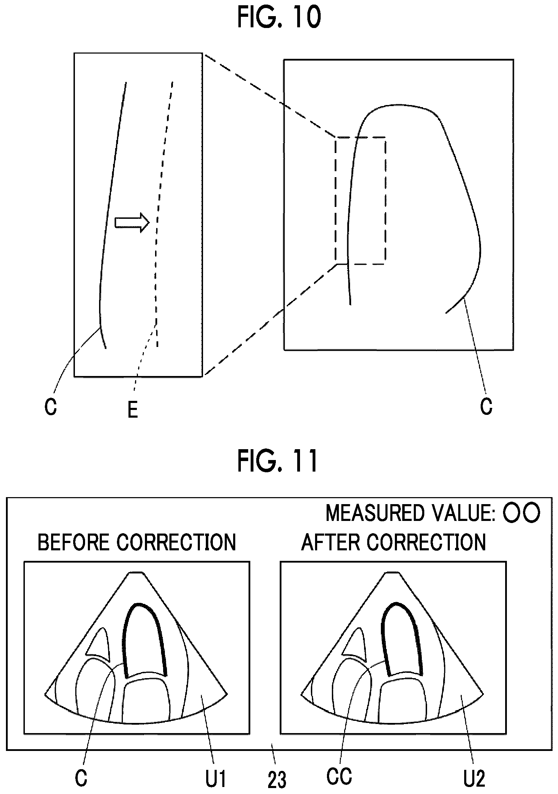

In addition, for example, the contour correction unit 26 can calculate an edge line E at which a brightness difference is maximized in the vicinity of the contour C of the cardiac chamber A extracted by the contour extraction unit 24 for the ultrasound image U of each frame as schematically shown in FIG. 10, and correct the contour C by deforming the contour C to align the contour C extracted by the contour extraction unit 24 with the edge line E.

In this way, since the contour C of the cardiac chamber A extracted by the contour extraction unit 24 is corrected by the contour correction unit 26, even in a case in which any of the contours C of the cardiac chamber A extracted by the contour extraction unit 24 deviates from the actual contour C of the cardiac chamber A, an accurate contour C can be finally obtained. Further, since both the processing of extracting the contour C via the contour extraction unit 24 and the processing of correcting the contour C via the contour correction unit 26 are completed only by the image analysis of the ultrasound image U, it is possible to obtain an accurate contour C without requiring a complicated apparatus configuration and processing.

The contour correction unit 26 stores the information on the contour C corrected in this way in the memory 25 in association with the ultrasound image U in which the contour C is shown.

The measurement target extraction unit 27 extracts the ultrasound image U, which is the target for measurement of the cardiac function, from the ultrasound images U of the plurality of frames based on the contour C of the cardiac chamber A extracted by the contour extraction unit 24. For example, in a case in which the ejection fraction of the cardiac chamber A is measured as the measurement of the cardiac function, the ultrasound image U of the frame corresponding to at least one of the end-systolic phase or the end-diastolic phase of the heart H is used as the target for measurement. In this case, for example, in a case in which the contour correction unit 26 acquires the cardiac phase information, the measurement target extraction unit 27 can extract the ultrasound image U of the frame corresponding to at least one of the end-systolic phase or the end-diastolic phase of the heart H as the target for measurement with reference to the cardiac phase information. In a case in which the contour correction unit 26 does not acquire the cardiac phase information, the measurement target extraction unit 27 can acquire the cardiac phase information using a method described as the method of acquiring the cardiac phase information via the contour correction unit 26.

The measurement unit 28 measures the cardiac function using the ultrasound image U which is extracted by the measurement target extraction unit 27 and on which the contour C of the cardiac chamber A corrected by the contour correction unit 26 is superimposed. For example, in a case of measuring the ejection fraction of the cardiac chamber A as the measurement of the cardiac function, the measurement unit 28 can calculate an area surrounded by the contour C of the cardiac chamber A in the ultrasound image U extracted by the measurement target extraction unit 27, and calculate the ejection fraction of the cardiac chamber A based on the calculated area.

The display controller 22 can display, for example, as shown in FIG. 11, an ultrasound image U1 on which the contour C extracted by the contour extraction unit 24 is superimposed, an ultrasound image U2 on which a contour CC corrected by the contour correction unit 26 is superimposed, and a measured value obtained by the measurement by the measurement unit 28 on the monitor 23 such that the user can check the correction result by the contour correction unit 26 and the measurement result by the measurement unit 28.

The display controller 22 can display, for example, the ultrasound image U2 designated by the user through the input device 30 and the ultrasound image U1 corresponding thereto among the ultrasound images U of the plurality of frames, on the monitor 23. In addition, the display controller 22 can also display the ultrasound image U2 extracted by the measurement target extraction unit 27 and the ultrasound image U1 corresponding to the ultrasound image U2, on the monitor 23. In this case, for example, the display controller 22 can display a list of the ultrasound images U2 having different cardiac cycles and the same phase on the monitor 23 as candidates for the ultrasound image U2 to be displayed, and allow the user to select the ultrasound image U2 displayed on the monitor 23 from a plurality of candidates for the ultrasound images U2.

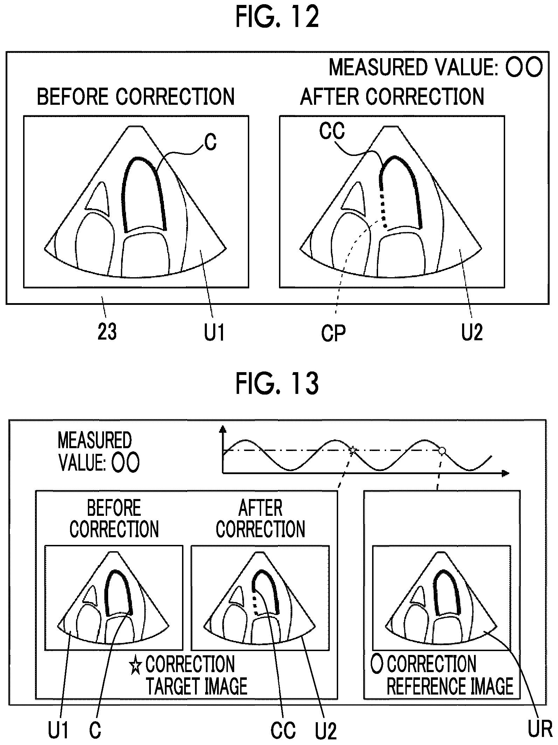

In addition, for example, as shown in FIG. 12, the display controller 22 can display a portion CP corrected by the contour correction unit 26 in the corrected contour CC in the ultrasound image U2 in an enhanced manner using a different display mode from the other portions of the contour CC. The display controller 22 can display the contour CC in an enhanced manner, for example, by displaying the portion CP corrected by the contour correction unit 26 with a broken line or the like, displaying the corrected portion CP in a color different from the surrounding color, displaying the corrected portion CP surrounded by a closed figure such as a circle, or displaying text such as "corrected portion" in the vicinity of the corrected portion CP. As a result, the user can easily grasp the specific correction portion in the contour CC.

In addition, in a case in which the contour C of the cardiac chamber A shown in the ultrasound image U of the frame that is the target for correction is corrected by the contour correction unit 26 based on the ultrasound image U of the frame before and after the ultrasound image U of the frame that is the target for correction, the ultrasound image U of the frame belonging to a cycle different from the cardiac cycle to which the ultrasound image U of the frame that is the target for correction belongs and having the same phase, or the like, the display controller 22 can display, for example, as shown in FIG. 13, the ultrasound image U of the frame different from the ultrasound image U of the target for correction, which is referred to in the correction of the contour C, as the correction reference image UR on the monitor 13.

In this case, the display controller 22 can display, on the monitor 23, the ultrasound image U on which the uncorrected contour C is superimposed and the ultrasound image U2 on which the corrected contour CC is superimposed, as correction target images.

It is possible for the user to easily check whether or not the contour CC is appropriately corrected by comparing the ultrasound image U2 on which the corrected contour CC is superimposed with the correction reference image UR.

In the present embodiment, each processing is executed by any computer. In addition, any computer may execute these pieces of processing by a processor as hardware, a program as software, or a combination thereof. In such a case, the processor is configured to execute various types of processing in the present embodiment in cooperation with the program, and can function as each unit or each means in the present embodiment. In addition, the execution order of the processing by the processor is not limited to the order described above and may be changed as appropriate. Any computer may be a general-purpose computer, a computer for a specific use, a workstation, or another system capable of executing each processing.

The processor 32 including the image generation unit 21, the display controller 22, the contour extraction unit 24, the contour correction unit 26, the measurement target extraction unit 27, the measurement unit 28, and the body controller 29 may be configured by one or a plurality of pieces of hardware, and the type of the hardware is not limited. For example, the processor 32 can be configured by a programmable logic device such as a central processing unit (CPU), a micro processing unit (MPU), or a field programmable gate array (FPGA), a dedicated circuit for executing specific processing, such as an application specific integrated circuit (ASIC), or hardware such as a graphic processing unit (GPU) or a neural processing unit (NPU). The types of hardware may be a combination of different types of hardware. In a case in which a plurality of types of hardware are configured to execute one or a plurality of types of processing of a certain processor, the plurality of types of hardware may be present in devices physically separated from each other or may be present in the same device. In any of the embodiments, the order of each processing performed by the processor is not limited to the above-described order, and may be changed as appropriate. Hardware is implemented in a form of an electric circuit (circuitry) in which circuit elements such as semiconductor elements are combined.

Further, the program may be software, such as firmware or a microcode. Further, the program may be, for example, a program module group, and each function thereof may be implemented by a processor configured to execute each function. The program may be a program code or a plurality of code segments stored in one or a plurality of non-transitory computer-readable media (for example, a storage medium or another storage). The program may be stored in a plurality of non-transitory computer-readable media present in devices physically separated from each other. The program code or the code segment can represent any combination of procedures, functions, subprograms, routines, subroutines, modules, software packages, classes, instructions, data structures, or program statements. The program code or the code segment may be connected to another code segment or a hardware circuit by transmitting and receiving information, data, arguments, parameters, or contents of the memory.

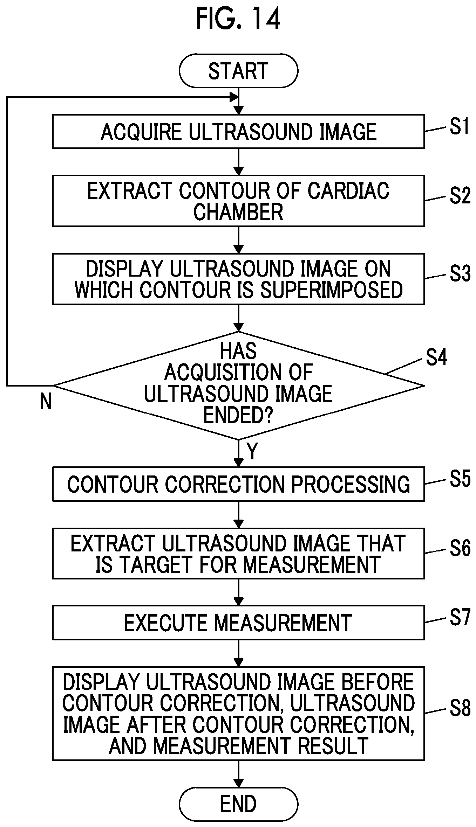

Hereinafter, an operation of the ultrasound diagnostic apparatus according to Embodiment 1 will be described with reference to a flowchart shown in FIG. 14.

In step S1, the image acquisition unit 31 generates the ultrasound image U in which the cardiac chamber A is imaged. In such a case, under the control of the body controller 29, the transmission and reception of the ultrasound from the plurality of transducers of the transducer array 11 are started in accordance with the drive signal from the pulser 41 of the transmission/reception circuit 12 of the ultrasound probe 1, the ultrasound echo from the subject is received by the plurality of transducers of the transducer array 11, and the reception signal as the analog signal is output to the amplifying unit 42, is amplified, and then is subjected to the AD conversion via the AD conversion unit 43 to acquire the reception data.

The reception focus processing is performed on the reception data by the beam former 44, the sound ray signal generated by the reception focusing processing is transmitted to the image generation unit 21 of the apparatus body 2, and thus the ultrasound image U representing the cardiac chamber A of the subject is generated by the image generation unit 21. In this case, the signal processing unit 45 of the image generation unit 21 performs the correction of the attenuation in accordance with the depth of the reflection position of the ultrasound and the envelope detection processing on the sound ray signal, the DSC 46 performs the conversion into the image signal in accordance with the normal television signal scanning method, and the image processing unit 47 performs various types of necessary image processing, such as gradation processing. The ultrasound image U generated in step S1 as described above is transmitted to the display controller 22, the contour extraction unit 24, and the memory 25.

In step S2, the contour extraction unit 24 extracts the contour C of the cardiac chamber A as shown in FIG. 4 from the ultrasound image U acquired in step S1. The contour extraction unit 24 can extract the contour C of the cardiac chamber A, for example, by using a trained model in machine learning, which has learned a relationship between a large number of ultrasound images U and the contour C of the cardiac chamber A shown therein. The information on the contour C extracted in step S2 is transmitted to the display controller 22 and the memory 25.

In addition, in step S2, the memory 25 stores the ultrasound image U acquired in step S1 and the information on the contour C of the cardiac chamber A extracted by the contour extraction unit 24 in association with each other.

In step S3, as shown in FIG. 4, the display controller 22 displays the ultrasound image U on which the contour C of the cardiac chamber A extracted in step S2 is superimposed, on the monitor 23 in real time.

In step S4, the body controller 29 determines whether or not to end the acquisition of the ultrasound image U. The body controller 29 can determine to end the acquisition of the ultrasound image U, for example, in a case in which an instruction to end the capturing of the ultrasound image U is input by the user via the input device 30. In addition, the body controller 29 can determine to continue the acquisition of the ultrasound image U in a case in which the instruction to end the capturing of the ultrasound image U is not particularly input from the user.

While it is determined in step S4 to continue the acquisition of the ultrasound image U, the processing of step S1 to step S4 is repeated. As a result, the ultrasound images U of the plurality of time-series consecutive frames corresponding to the plurality of cardiac beats are acquired, and the contour C of the cardiac chamber A is extracted in each ultrasound image U. The ultrasound images U of the plurality of frames obtained in this way and the information on the contour C of the cardiac chamber A extracted from the ultrasound images U are stored in the memory 25. Then, in a case in which it is determined in step S4 to end the acquisition of the ultrasound image U, the processing proceeds to step S5.

In step S5, the contour correction unit 26 performs the correction processing on the plurality of contours C of the cardiac chamber A such that the plurality of contours C of the cardiac chamber A extracted from each of the ultrasound images U of the plurality of frames acquired by repeating steps S1 to S4 and stored in the memory 25 smoothly change in time series.

For example, as shown in FIGS. 5 and 6, the contour correction unit 26 can correct the contour C2 of the cardiac chamber A in the ultrasound image U of the Nth frame extracted in step S2 such that the consistency with the contours C1 and C3 of the cardiac chamber A in the ultrasound images U of the (N-1)th and (N+1)th frames, which are the preceding and succeeding frames, is ensured.

For example, the contour correction unit 26 can acquire the cardiac phase information based on the changes in the plurality of contours C of the cardiac chamber A extracted in step S2, and correct the contour C of the cardiac chamber A by referring to the acquired cardiac phase information such that the positions of the contours C of the cardiac chamber A corresponding to the plurality of ultrasound images U having the same phase in different cardiac cycles are aligned with each other as shown in FIGS. 7 and 8.

For example, as shown in FIG. 9, the contour correction unit 26 can correct the contour C of the cardiac chamber A with reference to the cardiac phase information such that the positions of the structures of interest, such as the position of the cardiac apex D and the positions of the mitral valve bases M1 and M2, which are ideally not moved in the ultrasound image U by the cardiac beat, are aligned in the ultrasound images U having different cardiac cycles and the same phase.

For example, as shown in FIG. 10, the contour correction unit 26 can calculate the edge line E at which the brightness difference of the ultrasound image U is maximized in the vicinity of the contour C of the cardiac chamber A extracted in step S2, and correct the contour C such that the contour C overlaps the edge line E.

The information on the contour C corrected in step S5 in this manner is stored in the memory 25. Since the contour C of the cardiac chamber A extracted in step S2 is corrected in step S5 in this way, even in a case in which any of the contours C of the cardiac chamber A extracted in step S2 deviates from the actual contour C of the cardiac chamber A, an accurate contour C can be finally obtained. In addition, since both the processing of extracting the contour C of the cardiac chamber A in step S2 and the processing of correcting the contour C of the cardiac chamber A in step S5 are completed only by the image analysis of the ultrasound image U, it is possible to obtain an accurate contour C without requiring a complicated apparatus configuration and processing.

In subsequent step S6, the measurement target extraction unit 27 extracts the ultrasound image U, which is the target for measurement of the cardiac function, from among the ultrasound images U of the plurality of frames based on the contour C of the cardiac chamber A, which is extracted from the ultrasound images U of the plurality of frames in step S2 and is corrected in step S5. For example, in a case in which the ejection fraction of the cardiac chamber A is measured as the measurement of the cardiac function, the ultrasound image U of the frame corresponding to at least one of the end-systolic phase or the end-diastolic phase of the heart H is used as the target for measurement. In this case, the measurement target extraction unit 27 can extract the ultrasound image U of the frame corresponding to at least one of the end-systolic phase or the end-diastolic phase of the heart H as the target for measurement, for example, with reference to the cardiac phase information.

In step S7, the measurement unit 28 measures the cardiac function using the ultrasound image U extracted in step S6. For example, in a case of measuring the ejection fraction of the cardiac chamber A as the measurement of the cardiac function, the measurement unit 28 can calculate the area surrounded by the contour C of the cardiac chamber A in the ultrasound image U extracted in step S6, and calculate the ejection fraction of the cardiac chamber A based on the calculated area. The measurement result of the cardiac function obtained in step S7 is transmitted to the display controller 22 and the memory 25.

In a case in which any of the contours C of the cardiac chamber A is corrected in step S5, in step S8, for example, as shown in FIG. 11, the display controller 22 displays the ultrasound image U1 on which the contour C before correction extracted in step S2 is superimposed, the ultrasound image U2 on which the contour CC after correction by step S5 is superimposed, and the measurement result of step S7 on the monitor 23. The display controller 22 can display, for example, the ultrasound image U2 designated by the user through the input device 30 and the ultrasound image U1 corresponding thereto among the ultrasound images U of the plurality of frames, on the monitor 23. In addition, the display controller 22 can also display the ultrasound image U2 extracted by the measurement target extraction unit 27 and the ultrasound image U1 corresponding to the ultrasound image U2, on the monitor 23.

For example, as shown in FIG. 12, the display controller 22 can also display the portion CP corrected by the contour correction unit 26 in the corrected contour CC in the ultrasound image U2 in an enhanced manner using a different display mode from the other portions of the contour CC. As a result, the user can easily grasp the specific correction portion in the contour CC. In addition, in a case in which the contour C is not corrected in step S5, the display controller 22 can display only the ultrasound image U on which the contour C extracted in step S2 is superimposed, on the monitor 23.

In addition, in a case in which the contour C of the cardiac chamber A shown in the ultrasound image U of the frame that is the target for correction is corrected in step S5 based on the ultrasound image U of the frame before and after the ultrasound image U of the frame that is the target for correction, the ultrasound image U of the frame belonging to a cycle different from the cardiac cycle to which the ultrasound image U of the frame that is the target for correction belongs and having the same phase, or the like, the display controller 22 can display, for example, as shown in FIG. 13, the ultrasound image U of the frame different from the ultrasound image U of the frame to be corrected, which is referred to in the correction of the contour C, as the correction reference image UR on the monitor 13.

In this case, the display controller 22 can display, on the monitor 23, the ultrasound image U on which the uncorrected contour C is superimposed and the ultrasound image U2 on which the corrected contour CC is superimposed, as correction target images. It is possible for the user to easily check whether or not the contour CC is appropriately corrected by comparing the ultrasound image U2 on which the corrected contour CC is superimposed with the correction reference image UR.

In a case in which the processing of step S8 is completed in this way, the operation of the ultrasound diagnostic apparatus shown in FIG. 14 is completed.

As described above, with the ultrasound diagnostic apparatus according to Embodiment 1, the contour extraction unit 24 extracts the contour C of the cardiac chamber A from each of the ultrasound images U of the plurality of frames, the contour correction unit 26 performs the correction processing on the plurality of contours C of the cardiac chamber A extracted from the ultrasound images U of the plurality of frames such that the contour C of the cardiac chamber A smoothly changes in time series, and the display controller 22 displays the ultrasound image U on which the contour C of the cardiac chamber A extracted for each frame is superimposed, on the monitor 23 in real time, and then displays, in a case in which the contour C of the cardiac chamber A is corrected, the ultrasound image U on which the corrected contour CC of the cardiac chamber A is superimposed, on the monitor 23, so that it is possible to provide support for accurate measurement of the cardiac function with a simple apparatus configuration.

A case has been described in which the transmission/reception circuit 12 is provided in the ultrasound probe 1, but the transmission/reception circuit 12 may be provided in the apparatus body 2.

Further, a case has been described in which the image generation unit 21 is provided in the apparatus body 2, but the image generation unit 21 may be provided in the ultrasound probe 1.

The apparatus body 2 may be a so-called stationary type, a portable type that is easily carried, or a so-called handheld type that is configured by, for example, a smartphone or a tablet type computer. In this way, the type of the device constituting the apparatus body 2 is not particularly limited.

Although it has been described that the contour C of the cardiac chamber A in the ultrasound image U that is the target for correction is corrected by the contour correction unit 26 using the ultrasound images U of one frame before and after the ultrasound image U that is the target for correction, that is, a total of two ultrasound images U of one preceding frame and one succeeding frame, for example, the contour C of the cardiac chamber A can also be corrected using the ultrasound images U of two frames before and after the ultrasound image U that is the target for correction, that is, a total of four ultrasound images U of two preceding frames and two succeeding frames.

The number of the ultrasound images U to be referred to during the correction can also be changed between the diastolic phase and the systolic phase of the heart H. For example, since the left ventricle changes in shape more rapidly in the diastolic phase than in the systolic phase, the ultrasound images U of one preceding frame and one succeeding frame can be used in the diastolic phase, and the ultrasound images U of two preceding frames and two succeeding frames can be used in the systolic phase.

Although the example has been described in which the contour correction unit 26 corrects the contour C of the cardiac chamber A using the ultrasound images U of the three frames having different cardiac cycles and the same phase, the ultrasound images U of two frames having different cardiac cycles and the same phase can also be used, and the ultrasound images U of four or more frames having different cardiac cycles and the same phase can also be used.

In a case of extracting the contour C of the cardiac chamber A from the ultrasound image U, the contour extraction unit 24 can also calculate a reliability degree for each pixel on the contour C. The reliability degree is an indicator indicating a probability that the pixel accurately represents the contour C of the cardiac chamber A. A determination can be made that the higher the reliability degree, the more accurately the contour C of the cardiac chamber A is represented by the pixel, and a determination can be made that the lower the reliability degree, the less accurately the contour C of the cardiac chamber A is represented by the pixel. The contour extraction unit 24 can calculate the reliability degree by using, for example, a trained model in machine learning, which has been trained using, as training data, a relationship between a large number of ultrasound images U in which the cardiac chamber A is imaged, the contour C of the cardiac chamber A, and the reliability degree of each pixel on the contour C.

In a case in which the contour extraction unit 24 calculates the reliability degree for the pixels on the contour C of the cardiac chamber A, the contour correction unit 26 can also specify a low-reliability degree region in which the reliability degree is lower than a predetermined threshold value in each ultrasound image U, and correct only the contour C in the specified low-reliability degree region.

### Embodiment 2

In Embodiment 1, it has been described that the contour C of the cardiac chamber A is corrected after the acquisition of the ultrasound images U of the plurality of frames is completed, but the ultrasound diagnostic apparatus can also correct the contour C each time the contour C of the cardiac chamber A is extracted.

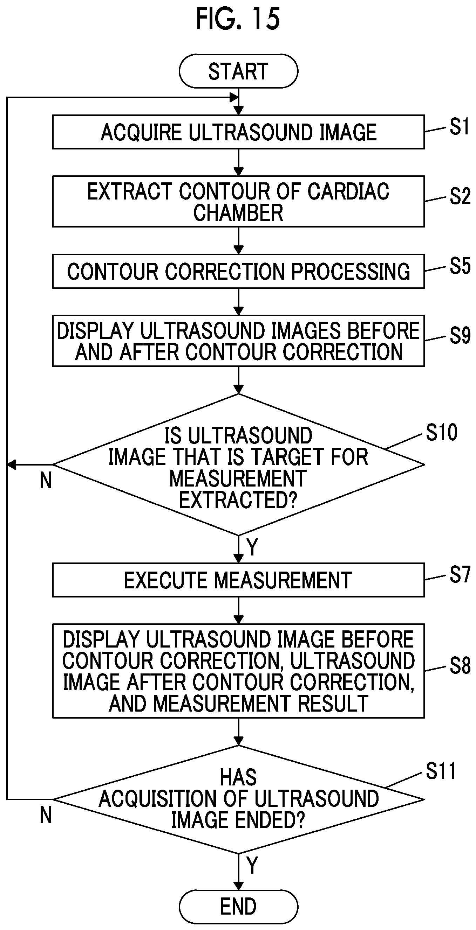

Since the ultrasound diagnostic apparatus according to Embodiment 2 has the same apparatus configuration as the ultrasound diagnostic apparatus according to Embodiment 1 shown in FIG. 1, the description of the apparatus configuration will be omitted, and the operation thereof will be described with reference to the flowchart of FIG. 15. The flowchart of FIG. 15 includes steps S1, S2, S5, S7, and S8 in the flowchart of FIG. 14 in Embodiment 1. Accordingly, detailed description of these steps will be omitted.

First, in step S1, the image acquisition unit 31 acquires the ultrasound image U in which the cardiac chamber A of the subject is imaged. In subsequent step S2, the contour extraction unit 24 extracts the contour C of the cardiac chamber A from the ultrasound image U acquired in step S1.

Next, in step S5, the contour correction unit 26 performs the correction processing on the contour C of the cardiac chamber A extracted in step S2. Since only the ultrasound image U of one frame is acquired at the current point in time, in a case in which the contour correction unit 26 performs the correction of the contour C using the plurality of ultrasound images U, such as the correction of the contour C using the ultrasound images U of the preceding and succeeding frames or the correction of the contour C using the ultrasound images U of frames having different cardiac cycles and the same phase, the processing of current step S5 can be skipped.

Next, in a case in which the contour C of the cardiac chamber A is corrected in step S5, in step S9, the display controller 22 displays the ultrasound image U on which the contour C of the cardiac chamber A extracted in step S2 is superimposed and the ultrasound image U on which the contour CC of the cardiac chamber A after being corrected in step S5 is superimposed, on the monitor 23. In a case in which the contour C is not corrected in step S5, the display controller 22 can display only the ultrasound image U on which the contour C extracted in step S2 is superimposed, on the monitor 23.

Next, in step S10, the measurement target extraction unit 27 performs the processing of extracting the ultrasound image U that is the target for measurement of the cardiac function on the ultrasound image U acquired up to the current point in time, and determines whether or not the ultrasound image U that is the target for measurement of the cardiac function can be extracted, in accordance with the extraction result. Since only the ultrasound image U of one frame is acquired at the current point in time, the ultrasound image U that is the target for measurement of the cardiac function is not extracted, and it is determined that the ultrasound image U that is the target for measurement of the cardiac function is not extracted.

While it is determined that the ultrasound image U that is the target for measurement of the cardiac function cannot be extracted, the processing of steps S1, S2, S5, S9, and S10 is repeated. In repeated step S1, the ultrasound images U of the plurality of frames are acquired. In addition, in repeated step S2, the contour C of the cardiac chamber A is extracted from each of the ultrasound images U of the plurality of frames. In addition, each time step S5 is performed, the correction processing is performed on the contour C of the cardiac chamber A in the ultrasound images U of the plurality of frames acquired so far. In step S5, an accurate contour C of the cardiac chamber A can be obtained.

In a case in which it is determined in step S10 that the ultrasound image U that is the target for measurement of the cardiac function can be extracted, the processing proceeds to step S7. In step S7, the measurement unit 28 measures the cardiac function based on the contour C of the cardiac chamber A in the ultrasound image U extracted in step S10.

In subsequent step S8, for example, as shown in FIG. 11, the display controller 22 displays the ultrasound image U1 on which the contour C of the cardiac chamber A extracted in step S2 is superimposed, the ultrasound image U2 on which the corrected contour CC obtained in step S5 is superimposed, and the measurement result of step S7 on the monitor 23.

In step S11, the body controller 29 determines whether or not to end the acquisition of the ultrasound image U. The body controller 29 can determine to end the acquisition of the ultrasound image U, for example, in a case in which the instruction to end the acquisition of the ultrasound image U is input by the user via the input device 30. In this case, the body controller 29 can determine to continue the acquisition of the ultrasound image U in a case in which the instruction to end the acquisition of the ultrasound image U is not particularly input by the user via the input device 30.

While it is determined in step S11 to continue the acquisition of the ultrasound image U, the processing of steps S1, S2, S5, S9, S10, S7, S8, and S11 is repeated. In a case in which it is determined in step S11 to end the acquisition of the ultrasound image U, the operation of the ultrasound diagnostic apparatus according to the flowchart of FIG. 15 is completed.

As described above, with the ultrasound diagnostic apparatus according to Embodiment 2, even in a case in which the contour correction unit 26 corrects the extracted contour C of the cardiac chamber A each time the contour C of the cardiac chamber A is extracted by the contour extraction unit 24, it is possible to provide support for accurate measurement of the cardiac function with a simple apparatus configuration.

### Embodiment 3

It has been described in Embodiments 1 and 2 that the processing of correcting the contour C of the cardiac chamber A is performed on all the ultrasound images U of the plurality of frames, but the ultrasound diagnostic apparatus can also correct only the ultrasound image U of a specific frame.

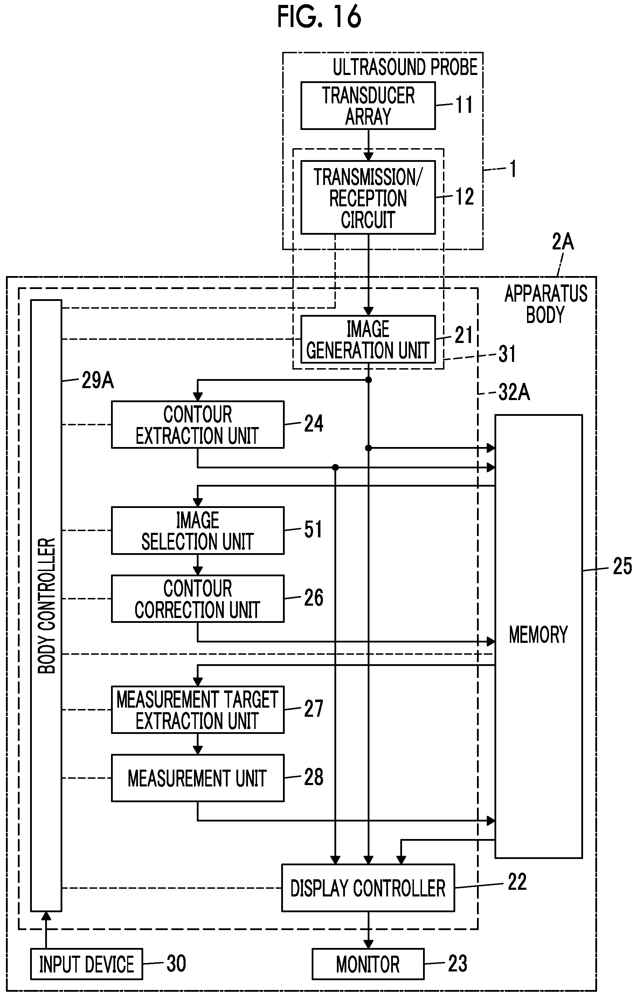

FIG. 16 shows a configuration of an ultrasound diagnostic apparatus according to Embodiment 3. The ultrasound diagnostic apparatus according to Embodiment 3 comprises an apparatus body 2A instead of the apparatus body 2 of the ultrasound diagnostic apparatus according to Embodiment 1 shown in FIG. 1. The apparatus body 2Ain Embodiment 3 further comprises an image selection unit 51 and comprises a body controller 29A instead of the body controller 29, as compared with the apparatus body 2 in Embodiment 1.

In the apparatus body 2A, the image selection unit 51 is connected to the memory 25. The contour correction unit 26 and the body controller 29A are connected to the image selection unit 51. In addition, the image generation unit 21, the display controller 22, the contour extraction unit 24, the contour correction unit 26, the measurement target extraction unit 27, the measurement unit 28, the body controller 29A, and the image selection unit 51 constitute a processor 32A for the apparatus body 2A.

The image selection unit 51 selects the ultrasound image U, which is the target for correction of the contour C of the cardiac chamber A via the contour correction unit 26, from among the ultrasound images U of the plurality of frames acquired by the image acquisition unit 31. The image selection unit 51 can select, as the target for correction, the ultrasound image U that may be extracted by the measurement target extraction unit 27 as the target for measurement of the cardiac function, such as the ultrasound image U corresponding to the end-diastolic phase or the end-systolic phase among the ultrasound images U of the plurality of frames.

For example, the image selection unit 51 can select the ultrasound image U selected by the user via the input device 30 as the target for correction.

The image selection unit 51 can acquire the cardiac phase information based on the contour C of the cardiac chamber A extracted by the contour extraction unit 24, and select the ultrasound image U that is the target for correction, such as the ultrasound image U corresponding to the end-diastolic phase or the end-systolic phase, based on the acquired cardiac phase information. The image selection unit 51 can calculate the volume of the cardiac chamber A based on the plurality of contours C of the cardiac chamber A extracted by the contour extraction unit 24 for the ultrasound images U of the plurality of frames, and acquire the cardiac phase information based on the time-series change in volume of the cardiac chamber A, for example, in the same manner as the measurement target extraction unit 27.

The contour correction unit 26 performs the correction processing on the contour C of the cardiac chamber A in the ultrasound image U selected as the target for correction by the image selection unit 51. The contour correction unit 26 corrects only the ultrasound image U selected by the image selection unit 51 instead of correcting the contour C of the cardiac chamber A in the ultrasound images U of all the frames acquired by the image acquisition unit 31, so that the calculation load on the contour correction unit 26 can be reduced, and the correction processing of the contour C can be completed more quickly.

As described above, with the ultrasound diagnostic apparatus according to Embodiment 3, the image selection unit 51 selects the ultrasound image U that is the target for correction of the contour C of the cardiac chamber A via the contour correction unit 26 from among the ultrasound images U of the plurality of frames, and the contour correction unit 26 performs the correction processing of the contour C of the cardiac chamber A on the ultrasound image U selected by the image selection unit 51, so that the calculation load on the contour correction unit 26 can be reduced, and the measurement of the cardiac function can be completed more quickly.

In addition, in a case in which the user selects a new ultrasound image U as the target for correction via the input device 30 after the display controller 22 displays the ultrasound image U on which the corrected contour C of the cardiac chamber A is superimposed, on the monitor 23, the contour correction unit 26 can correct the contour C of the cardiac chamber A again. The contour C of the cardiac chamber A in the ultrasound image U selected by the image selection unit 51 is corrected by the contour correction unit 26, and thus it may be found that the ultrasound image U is appropriate as the target for measurement in another ultrasound image U. Therefore, the measurement unit 28 can accurately measure the cardiac function by correcting the contour C of the cardiac chamber A in the ultrasound image U.

### Explanation of References

1: ultrasound probe
2, 2A: apparatus body
11: transducer array
12: transmission/reception circuit
21: image generation unit
22: display controller
23: monitor
24: contour extraction unit
25: memory
26: contour correction unit
27: measurement target extraction unit
28: measurement unit
29, 29A: body controller
30: input device
31: image acquisition unit
32, 32A: processor
41: pulser
42: amplifying unit
43: AD conversion unit
44: beam former
45: signal processing unit
46: DSC
47: image processing unit
51: image selection unit
4C: apical four-chamber cross section
A: cardiac chamber
C, C1 to C6, CC: contour
CP: portion
D: cardiac apex
E: edge line
M1, M2: mitral valve base
U, U1, U2: ultrasound image
UR: correction reference image

## Claims

1. An ultrasound diagnostic apparatus comprising:
an ultrasound probe (1);
a monitor (23);
an image acquisition unit (31) that acquires ultrasound images of a plurality of frames as a moving image in which a heart of a subject is imaged, by transmitting and receiving ultrasound beams using the ultrasound probe (1);
a contour extraction unit (24) that extracts a contour of a cardiac chamber from each of the ultrasound images of the plurality of frames acquired by the image acquisition unit (31);
a contour correction unit (26) that performs correction processing on a plurality of contours of the cardiac chamber extracted by the contour extraction unit (24) from the ultrasound images of the plurality of frames such that the plurality of contours of the cardiac chamber smoothly change in time series; and
a display controller (22) that displays the ultrasound image on which the contour of the cardiac chamber extracted by the contour extraction unit (24) for each frame is superimposed, on the monitor (23) in real time, and that displays the ultrasound image on which the contour of the cardiac chamber on which the correction processing is performed by the contour correction unit (26) is superimposed, on the monitor (23).

2. The ultrasound diagnostic apparatus according to claim 1, further comprising:
a measurement target extraction unit (27) that extracts the ultrasound image, which is a target for measurement, from among the ultrasound images of the plurality of frames based on the contour of the cardiac chamber extracted by the contour extraction unit (24).

3. The ultrasound diagnostic apparatus according to claim 1 or 2,
wherein the contour correction unit (26) corrects the contour of the cardiac chamber extracted by the contour extraction unit (24) such that consistency with the contour of the cardiac chamber in preceding and succeeding frames is ensured.

4. The ultrasound diagnostic apparatus according to any one of the previous claims, in particular claim 1 or 2,
wherein the contour correction unit (26) corrects the contour of the cardiac chamber based on positional information of a characteristic structure of the cardiac chamber.

5. The ultrasound diagnostic apparatus according to any one of the previous claims, in particular claim 1 or 2,
wherein the contour correction unit (26)
acquires cardiac phase information based on the contour of the cardiac chamber extracted by the contour extraction unit (24), and
corrects the contour of the cardiac chamber by referring to the cardiac phase information such that positions of the contour of the cardiac chamber corresponding to a plurality of ultrasound images having the same phase in different cardiac cycles are aligned with each other.

6. The ultrasound diagnostic apparatus according to any one of the previous claims, in particular claim 1 or 2,
wherein the contour correction unit (26)
calculates an edge line at which a brightness difference is maximized in a vicinity of the contour of the cardiac chamber extracted by the contour extraction unit (24), and
corrects the contour of the cardiac chamber such that the contour of the cardiac chamber is aligned with the edge line.

7. The ultrasound diagnostic apparatus according to any one of claims 1 to 6,
wherein the display controller (22) displays a portion of the ultrasound image related to correction performed by the contour correction unit (26) in an enhanced manner.

8. The ultrasound diagnostic apparatus according to any one of the previous claims, in particular any one of claims 1 to 6, further comprising:
an image selection unit (51) that selects the ultrasound image, which is a target for correction of the contour of the cardiac chamber via the contour correction unit (26), from among the ultrasound images of the plurality of frames.

9. The ultrasound diagnostic apparatus according to any one of the previous claims, in particular claim 8, further comprising:
an input device (30) for a user to perform an input operation,
wherein the image selection unit (51) selects the ultrasound image selected by the user via the input device (30) as the target for the correction.

10. The ultrasound diagnostic apparatus according to any one of the previous claims, in particular claim 9,
wherein in a case in which the user selects a new ultrasound image as the target for the correction via the input device (30) after the display controller (22) displays the ultrasound image on which the corrected contour of the cardiac chamber is superimposed, on the monitor (23), the contour correction unit (26) corrects the contour of the cardiac chamber again.

11. The ultrasound diagnostic apparatus according to any one of the previous claims, in particular claim 8,
wherein the image selection unit (51)
acquires cardiac phase information based on the contour of the cardiac chamber extracted by the contour extraction unit (24), and
selects the ultrasound image, which is the target for the correction, based on the acquired cardiac phase information.

12. The ultrasound diagnostic apparatus according to any one of the previous claims, in particular claim 2, further comprising:
a measurement unit that measures a cardiac function using the ultrasound image which is the target for the measurement extracted by the measurement target extraction unit (27) and on which the contour of the cardiac chamber corrected by the contour correction unit (26) is superimposed.

13. The ultrasound diagnostic apparatus according to any one of the previous claims, in particular claim 12,
wherein the display controller (22) displays a measurement result of the measurement unit (28) on the monitor (23) together with the ultrasound image on which the contour of the cardiac chamber extracted by the contour extraction unit (24) is superimposed.

14. The ultrasound diagnostic apparatus according to any one of claims 1 to 13,
wherein the contour correction unit (26) corrects the extracted contour of the cardiac chamber each time the contour of the cardiac chamber is extracted by the contour extraction unit (24).

15. A method of controlling an ultrasound diagnostic apparatus, the method comprising:
acquiring ultrasound images of a plurality of frames as a moving image in which a heart of a subject is imaged, by transmitting and receiving ultrasound beams using an ultrasound probe (1);
extracting a contour of a cardiac chamber from each of the acquired ultrasound images of the plurality of frames;
performing correction processing on a plurality of contours of the cardiac chamber extracted from the ultrasound images of the plurality of frames such that the plurality of contours of the cardiac chamber smoothly change in time series; and
displaying the ultrasound image on which the contour of the cardiac chamber extracted for each frame is superimposed, on a monitor (23) in real time, and displaying the ultrasound image on which the contour of the cardiac chamber on which the correction processing is performed is superimposed, on the monitor (23).
